# EUROPEAN PATENT APPLICATION

(11) **EP 2 653 193 A1**
(43) Date of publication of application: **23.10.2013**
(21) Application number: 13176594.3
(22) Date of filing: 18.12.2009
(51) Int. Cl.: A61P 27/02, A61K 31/505

(54) **Method for treating macular degeneration**

(30) Priority: 18.12.2008 US 138705 P; 25.08.2009 FR 0955794
(62) Divisional of application: 09796895.2
(71) Applicant: SANOFI, 75008 Paris (FR)
(72) Inventor: Hahn, Chang S., Bridgewater, NJ New Jersey 08807 (US)
(74) Representative: Zwicker, Jörk

(57) **Abstract**

Provided herein are novel and useful methods for preventing, treating, r ameliorating a macular degeneration such as dry macular degeneration and age related macular degeneration in a patient.

## Description

### FIELD OF THE INVENTION

The present invention relates to a method for treating, preventing or ameliorating macular degeneration in a patient

### BACKGROUND OF THE INVENTION

Macular degeneration is the general term for a disorder in which a part of the retina called the macula deteriorates. Age-related macular degeneration (AMD) is the most common type of macular degeneration. It has been reported that in the United States, AMD is the leading cause of blindness in people older than 55. More than 10 million people in the US are affected by this disease, which includes 23% of people over 90. (www.webmd.com/eye-health/macular-degeneration/macular-degeneration-overview).

There are various types of macular degeneration that afflict patients. One type of macular degeneration is "dry" macular degeneration, also called geographical atrophy. Dry macular degeneration is an early stage of the disorder in which drusen is deposited on the macula at a subretinal level. The deposition of this drusen may result from aging or thinning of the macular tissues. As a result of this deposition of drusen, loss of central vision may gradually occur. Many times, AMD begins with dry macular degeneration.

Another type of AMD is "wet" macular degeneration. Wet macular degeneration is a neovascular type of degeneration in which incomplete blood vessels abnormally grow under the retina and begin to leak. As a result of this leakage, permanent damage occurs to photoreceptor cells of the retina which ultimate causes the death of these cells and thus, blind spots. Unlike dry macular degeneration, in which the vision loss may be minor, the vision loss that occurs in wet macular degeneration can be severe. Indeed, it has been reported that although only 10% of those with AMD suffer from wet macular degeneration, 66% of those with AMD suffering from significant visual loss can directly attribute that loss to wet macular degeneration.

Unfortunately, there has only been limited success determining the causes for the disorder. Moreover, treatments for macular degeneration have met with only limited success. To date, there is no FDA-approved treatment for dry macular degeneration and nutritional intervention is used to prevent the progression of wet macular degeneration. Also, treatment of wet macular degeneration is limited to intravitreal injection of compounds that are anti-angiogenic in nature.

Accordingly, what is needed is a method for preventing, treating, or ameliorating macular degeneration.

The citation of any reference herein should not be construed as an admission that such reference is available as "Prior Art" to the instant application.

### SUMMARY OF THE INVENTION

Provided herein are heretofore unknown methods for preventing, treating, or ameliorating various forms of macular degeneration as well as a heretofore unknown method for treating choroidal neovascularization, which in turn readily has applications in treating macular degeneration.

Broadly, the present invention extends to a method for treating, preventing or ameliorating choroidal neovascularization in a patient comprising administering to the patient an effective amount of a compound that modulates the patient's immune system, wherein the immune system has type I immune responses and type II immune responses, such that administering the compound to the patient increases the type I immune responses in the patient as compared to the type I immune responses in the patient prior to administering of the compound.

The present invention further extends to a method for treating, ameliorating or preventing macular degeneration in a patient, comprising administering to the patient an effective amount of a compound that modulates the patient's immune system, wherein the immune system has type I immune responses and type II immune responses, such that administering the compound to the patient increases the type I immune responses in the patient as compared to the type I immune responses in the patient prior to administering of the compound.

In addition, the present invention extends to a method for treating, preventing or ameliorating macular degeneration in a patient, comprising administering to the patient an effective amount of a compound that modulates the patient's immune system, wherein the immune system has type I immune responses and type II immune responses, such that administering the compound to the patient increases the type I immune responses in the patient as compared to the type I immune responses in the patient prior to administering of the compound.

The present invention also extends to a method of treating or ameliorating macular degeneration in a patient having an immune system which has type I immune responses and type II immune responses, the method comprising administering to the patient an effective amount of a compound that modulates the activity of an immunocyte in the patient, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof, which increases the type I immune responses in the patient relative to the type I immune responses in the patient prior to administering the compound, and the increase in the type I responses treats, ameliorates or prevents choroidal neovascularization in the patient.

In another embodiment, the present invention extends to a method of treating or ameliorating wet macular degeneration in a patient having an immune system which has type I immune responses and type II immune responses, the method comprising administering to the patient an effective amount of a compound that modulates the activity of an immunocyte in the patient, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof, so that the type I immune responses in the patient are increased relative to the type I immune responses in the patient prior to administering the compound, and the increase in the type I responses ameliorates or prevents choroidal neovascularization in the patient, wherein the compound is selected from the group consisting of:
(a) phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt 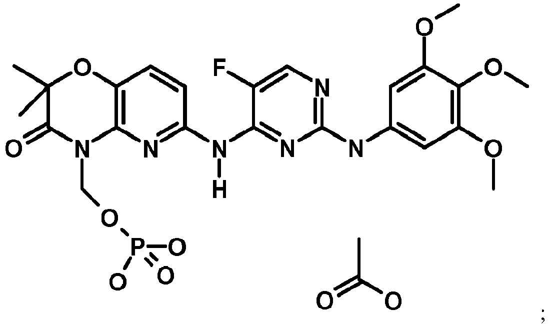
(b) 2-[4-(7-Ethyl-5H-pyrrolo[2,3-b]pyrazin-6-yl)-phenyl]-propan-2-ol 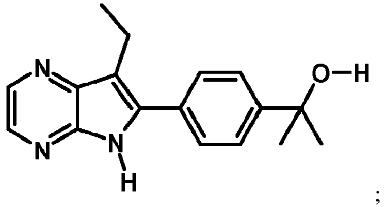
(c) 2-(3-Fluoro-phenyl)-4-methyl-pyrimidine-5-carboxylic acid indol-1-ylamide 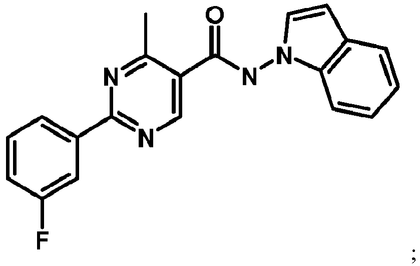 and
(d) 2-(3-{6-[2-(2,4-Dichloro-phenyl)-ethylamino]-2-methoxy-pyrimidin-4-yl}-phenyl)-2-methyl-propionic Acid Phosphoric Acid Salt 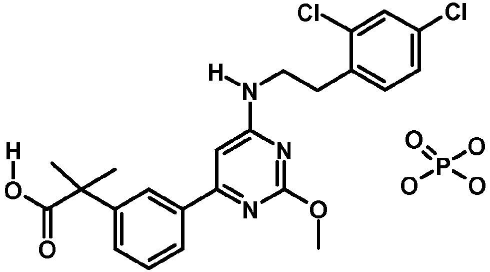
(e) 2-Pyridin-2-yl-pyrimidine-5-carboxylic acid 3-[5-(1-hydroxy-1-methyl-ethyl)-1,2,4-oxadiazol-3-yl]-benzylamide 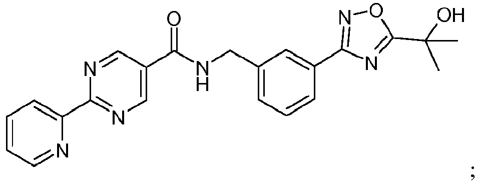 and
(f) 4-Methyl-2-pyridin-2-yl-pyrimidine-5-carboxylic acid (5-fluoro-3-methyl-indol-1-yl)-amide 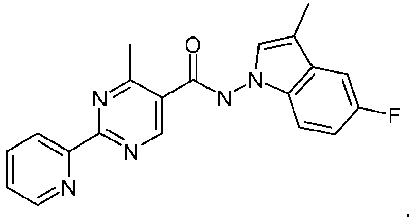
2-Pyridin-2-yl-pyrimidine-5-carboxylic acid 3-[5-(1-hydroxy-1-methyl-ethyl)-1,2,4-oxadiazol-3-yl]-benzylamide is disclosed in U.S. Provisional Application 61/249,963 filed October 8, 2009, which is hereby incorporated by reference herein in its entirety. In addition, other
2-(3-Fluoro-phenyl)-4-methyl-pyrimidine-5-carboxylic acid indol-1-ylamide is disclosed in published PCT application WO2009/114373, which is hereby incorporated by reference herein in its entirety.

The present invention still further extends to a method of preventing or ameliorating macular degeneration in a patient having an immune system which has type I immune responses and type II immune responses, the method comprising administering to the patient an effective amount of a compound that modulates the activity of an immunocyte in the patient, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof, so that the type I immune responses in the patient are increased relative to the type I immune responses in the patient prior to administering the compound, and the increase in the type I responses ameliorates or prevents choroidal neovascularization in the patient, wherein the compound is selected from the group consisting of:
(a) phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt;
(b) 2-[4-(7-ethyl-5H-pyrrolo[2,3-b]pyrazin-6-yl)-phenyl]-propan-2-ol;
(c) 2-(3-fluoro-phenyl)-4-methyl-pyrimidine-5-carboxylic acid indol-1-ylamide;
(d) 2-(3-{6-[2-(2,4-dichloro-phenyl)-ethylamino]-2-methoxy-pyrimidin-4-yl}-phenyl)-2-methyl-propionic acid phosphoric acid salt;
(e) 2-Pyridin-2-yl-pyrimidine-5-carboxylic acid 3-[5-(1-hydroxy-1-methyl-ethyl)-1,2,4-oxadiazol-3-yl]-benzylamide ; and
(f) 4-Methyl-2-pyridin-2-yl-pyrimidine-5-carboxylic acid (5-fluoro-3-methyl-indol-1-yl)-amide.

Naturally, a method of the present invention readily has applications in treating any type of macular degeneration, including, but certainly not limited to dry macular degeneration, wet macular degeneration, and age-related macular degeneration.

Moreover, numerous types of compounds have applications in a method of the present invention. Examples of such types that readily have applications in a method of the present invention are (a) a syk multikinase inhibitor, an hPGDS inhibitor; and a DP antagonist, to name only a few.

Syk multikinase inhibitors having applications in a method of the present invention are clearly set forth in PCT Published Patent Application WO 2003035065, which is hereby incorporated by reference in its entirety. Particular examples of syk multikinase inhibitors having applications in a method of the present invention are phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt and 2-[4-(7-ethyl-5H-pyrrolo[2,3-b]pyrazin-6-yl)-phenyl]-propan-2-ol, to name only a few. Other examples of syk multikinase inhibitors having applications in a method of the present invention are set forth in U.S. Patent 7,449,458 which is hereby incorporated by reference herein in its entirety.

hPGDS inhibitors also readily have applications in a method of the present invention. Particular hPGDS inhibitors having applications herein are 2-(3-fluoro-phenyl)-4-methyl-pyrimidine-5-carboxylic acid indol-1-ylamide, 2-Pyridin-2-yl-pyrimidine-5-carboxylic acid 3-[5-(1-hydroxy-1-methyl-ethyl)-1,2,4-oxadiazol-3-yl]-benzylamide, and 4-Methyl-2-pyridin-2-yl-pyrimidine-5-carboxylic acid (5-fluoro-3-methyl-indol-1-yl)-amide, to name only a few. Moreover, additional hPGDS inhibitors that readily have applications in the present invention are disclosed in U.S. patent applications 12/062,641 and 12/570,355, which are hereby incorporated by reference in their entireties.

Furthermore, a DP antagonist such as 2-(3-{6-[2-(2,4-dichloro-phenyl)-ethylamino]-2-methoxy-pyrimidin-4-yl}-phenyl)-2-methyl-propionic acid phosphoric acid salt, for example also readily have applications in a method of the present invention.

Furthermore, in a method of the present invention, administration of a compound as described herein to a patient suffering from macular degeneration modulates the activity of an immunocyte in the patient. The activity of numerous types of immunocytes can be modulated in a method of the present invention. Examples of such immunocytes include a natural killer cell (NK cell), a natural killer T cell (NKT cell), a mast cell, a dendritic cell, and granulocyte selected from the group consisting of an eosinophil, a basophil and neutrophil. Naturally, the activity of a combination of these cells can also be modulated in a method of the present invention.

Moreover, a method of the present invention can also be used to treat or ameliorate choroidal neovascularization, which in turn also treats or ameliorates wet macular degeneration in a patient.

These and other aspects of a method of the present invention will be better appreciated by reference to the following drawings and Detailed Description.

### BRIEF DESCRIPTION OF THE DRAWINGS

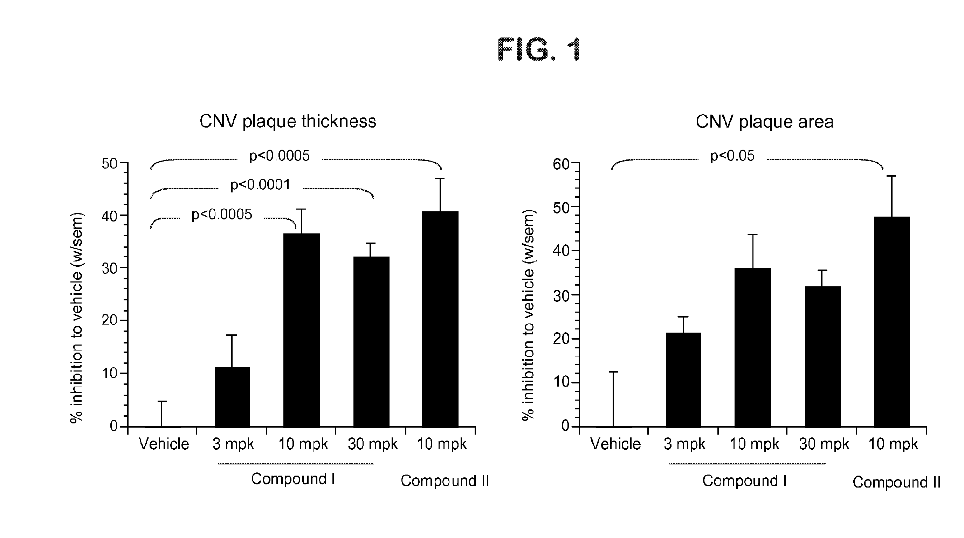

FIG. 1 is a demonstration of a reduction of choroidal neovascular plaque thickness and area reduction upon treatment with a compound when compared to that of vehicle treatment. "Compound I" is 2-[4-(7-Ethyl-5H-pyrrolo[2,3-b]pyrazin-6-yl)-phenyl]-propan-2-ol. "Compound II" is 2-(3-Fluoro-phenyl)-4-methyl-pyrimidine-5-carboxylic acid indol-1-ylamide.

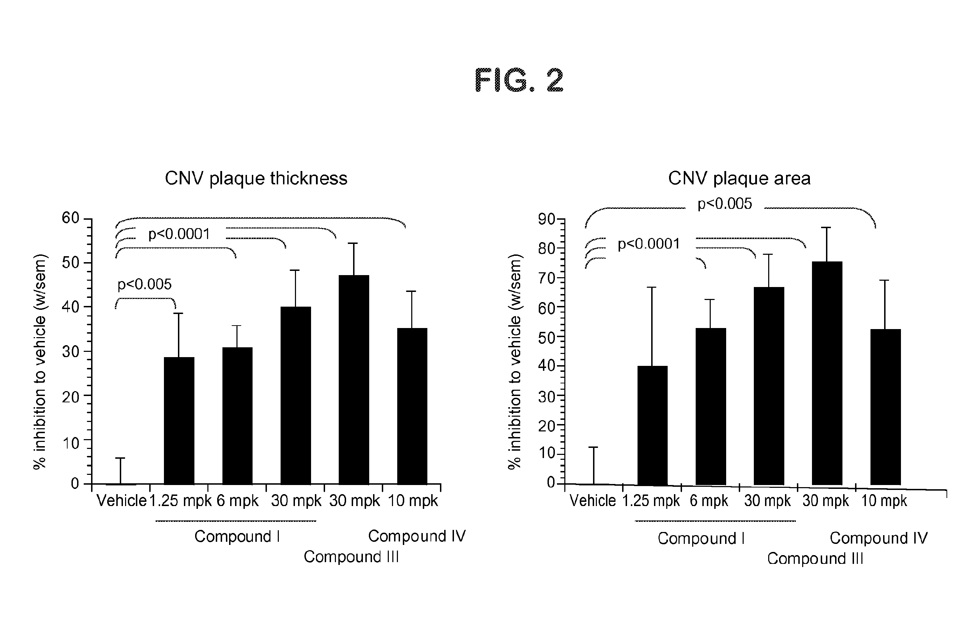

FIG. 2 is a demonstration of a reduction of choroidal neovascular plaque thickness and area reduction upon treatment with a compound when compared to that of vehicle treatment. "Compound I" is 2-[4-(7-Ethyl-5H-pyrrolo[2,3-b]pyrazin-6-yl)-phenyl]-propan-2-ol. "Compound III" is phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt. "Compound IV" is 2-(3-{6-[2-(2,4-Dichloro-phenyl)-ethylamino]-2-methoxy-pyrimidin-4-yl}-phenyl)-2-methyl-propionic Acid Phosphoric Acid Salt.

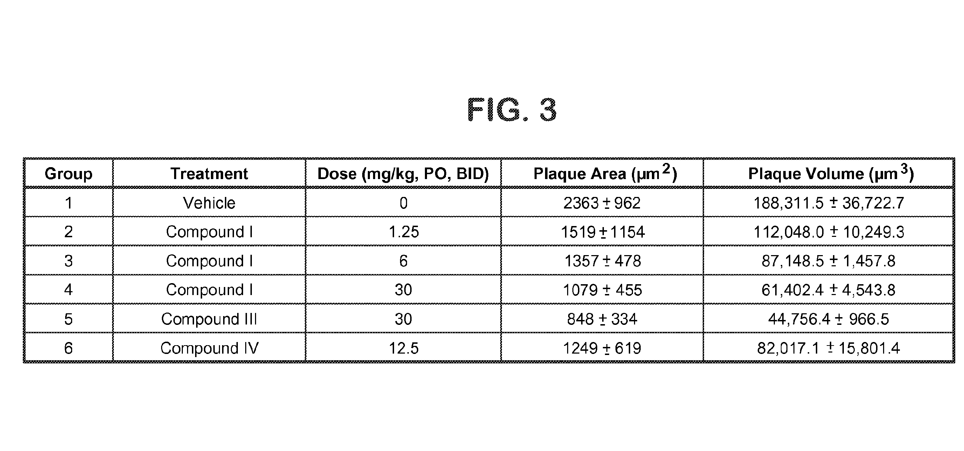

FIG. 3 is a demonstration of a reduction of choroidal neovascular plaque area and volume upon treatment with a compound when compared to that of vehicle treatment. For purposes of this figure, "Compound I" is 2-[4-(7-Ethyl-5H-pyrrolo[2,3-b]pyrazin-6-yl)-phenyl]-propan-2-ol; "Compound III" is phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt; and "Compound IV" is 2-(3-{6-[2-(2,4-Dichloro-phenyl)-ethylamino]-2-methoxy-pyrimidin-4-yl}-phenyl)-2-methyl-propionic Acid Phosphoric Acid Salt.

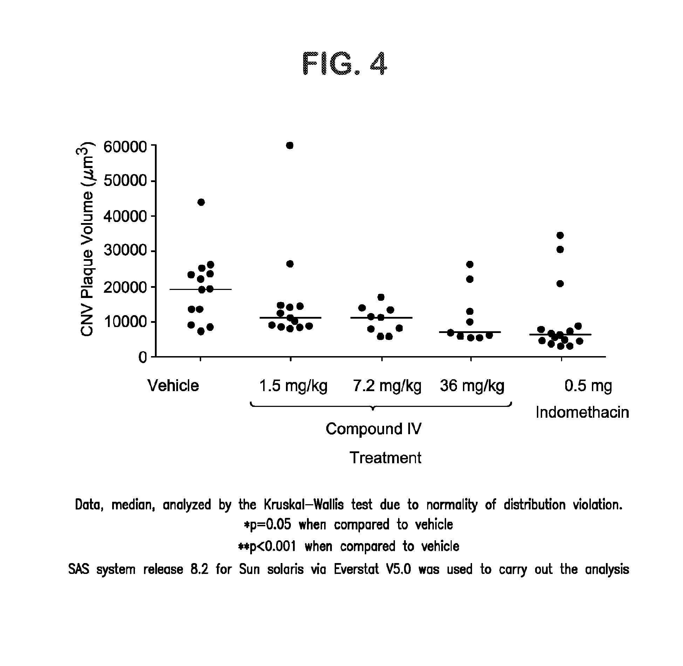

FIG. 4 is a histogram of the results of an analysis of the effect of DP antagonist 2-(3-{6-[2-(2,4-dichloro-phenyl)-ethylamino]-2-methoxy-pyrimidin-4-yl}-phenyl)-2-methyl-propionic acid phosphoric acid salt ("Compound V" for purposes of this figure) on the plaque volume of choroidal neovascularization in the rat laser-induced macular degeneration model. Indomethacin was used as a positive control.

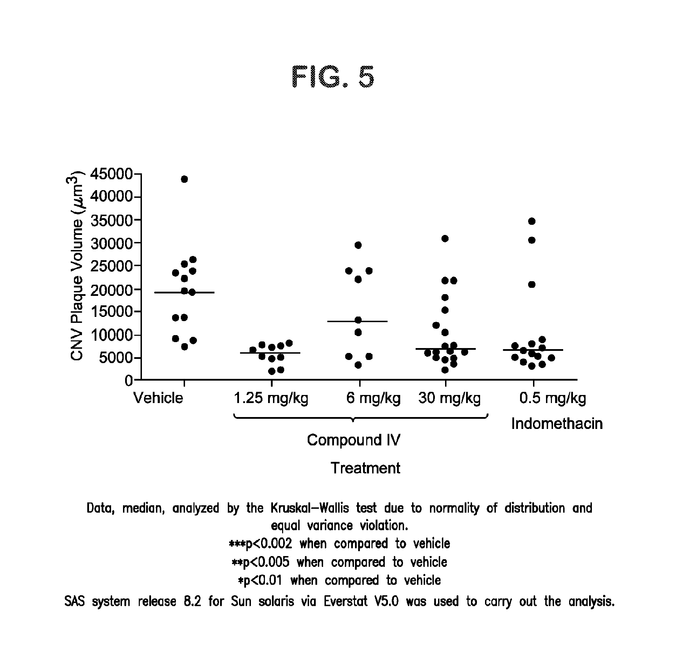

FIG. 5 is a histogram of the results of an analysis of the effect of the hPDGS inhibitor 4-Methyl-2-pyridin-2-yl-pyrimidine-5-carboxylic acid (5-fluoro-3-methyl-indol-1-yl)-amide ("Compound VI" for purposes of this figure) on the plaque volume of choroidal neovascularization in the rat laser-induced macular degeneration model. Indomethacin was used as a positive control.

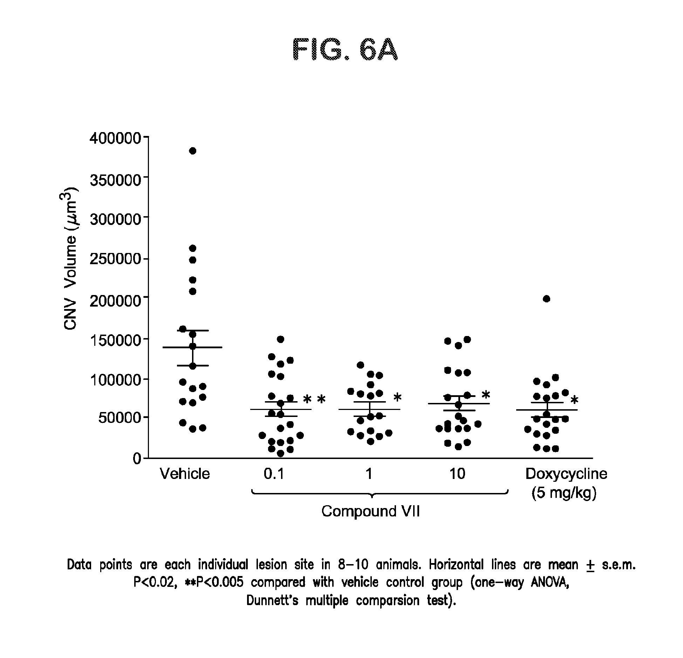

FIG. 6A is a histogram of the results of the effect of the hPDGS inhibitor 2-Pyridin-2-yl-pyrimidine-5-carboxylic acid 3-[5-(1-hydroxy-1-methyl-ethyl)-1,2,4-oxadiazol-3-yl]-benzylamide ("Compound VII" for purposes of this figure) against a laser-induced choroidal neovascularization in the rat: 1^{st} dose-response study. 2-Pyridin-2-yl-pyrimidine-5-carboxylic acid 3-[5-(1-hydroxy-1-methyl-ethyl)-1,2,4-oxadiazol-3-yl]-benzylamide was administered orally twice each day on days -1 to 7. Doxycycline was used as a positive control.

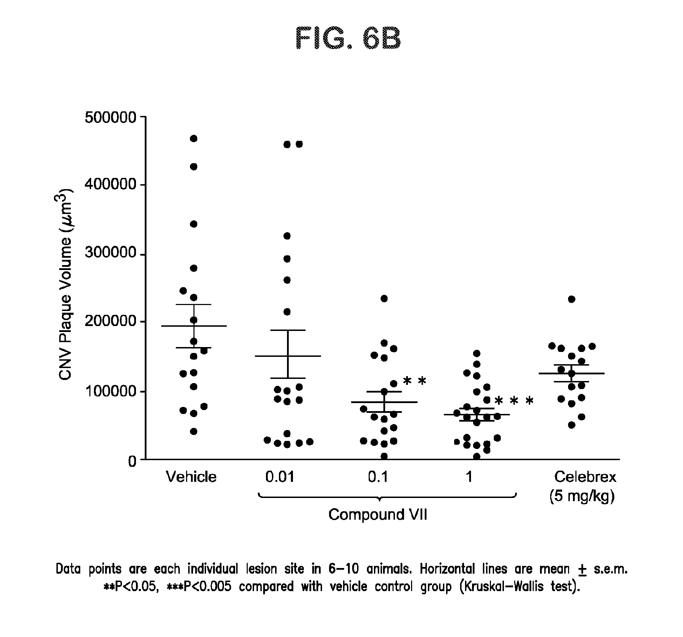

FIG. 6B is a histogram of the results of the effect of the hPDGS inhibitor 2-pyridin-2-yl-pyrimidine-5-carboxylic acid 3-[5-(1-hydroxy-1-methyl-ethyl)-1,2,4-oxadiazol-3-yl]-benzylamide ("Compound VII" for purposes of this figure) against a laser-induced choroidal neovascularization in the rat: 2nd dose-response study. 2-pyridine-2-yl-pyrimidine-5-carboxylic acid 3-[5-(1-hydroxy-1-methyl-ethyl)-1,2,4-oxadiazol-3-yl]-benzylamide was administered orally twice each day on days -1 to 7. CELEBREX was used as a positive control.

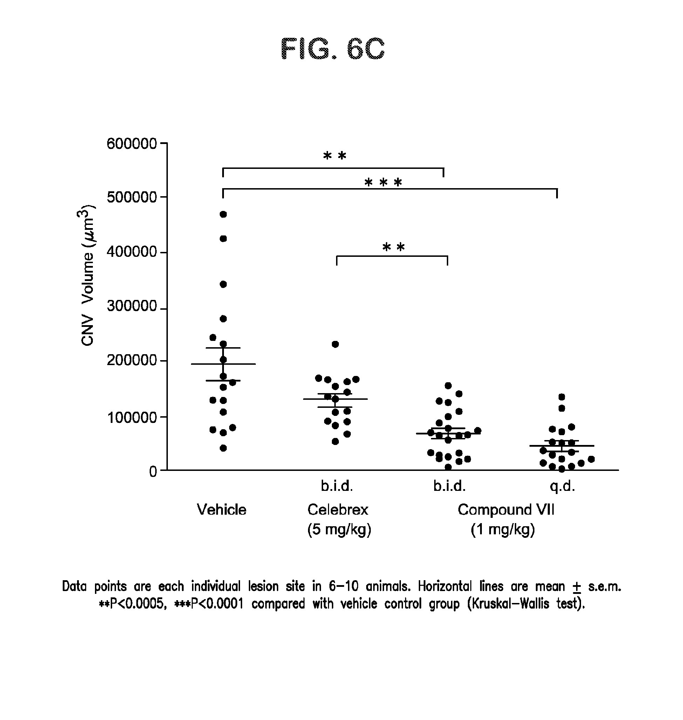

FIG. 6C is a histogram of the effect of hPDGS inhibitor 2-pyridin-2-yl-pyrimidine-5-carboxylic acid 3-[5-(1-hydroxy-1-methyl-ethyl)-1,2,4-oxadiazol-3-yl]-benzylamide ("Compound VII" for purposes of this figure) against choroidal neovascularization in the rat: once-daily dosing. In this experiment Brown Norway rats received laser coagulation on day 0, and 2-pyridin-2-yl-pyrimidine-5-carboxylic acid 3-[5-(1-hydroxy-1-methyl-ethyl)-1,2,4-oxadiazol-3-yl]-benzylamide was administered orally once (q.d.) or twice (b.i.d.) each day on days -1 to 7. CELEBREX was used as a positive control.

### DETAILED DESCRIPTION OF THE INVENTION

The present invention is based upon the novel and useful discovery that altering or modulating immunocytes involved in dictating whether the immune responses deviates toward either type I or type II immune responses during transition from innate immune responses to the adapted immune responses of activities of these cell types, including lymphocytes, macrophages, monocytes, NK cells, NKT cells, mast cells and dendritic cells in order to increase the type I immune responses, will modulate the development and progression of choroidal neovascularization (CNV), the hallmark of the wet form of the age-related macular degeneration (AMD). Thus administering a compound that modulates the activity of immunocytes so as to increase the activity of the type I immune responses readily can be used in a method to treat macular degeneration.

Hence broadly, the present invention extends to a method for preventing treating or ameliorating choroidal neovascularization in a patient comprising administering to the patient an effective amount of a compound that modulates the patient's immune system, wherein the immune system has type I immune responses and type II immune responses, such that administering the compound to the patient increases the type I immune responses in the patient as compared to the type I immune responses in the patient prior to administering of the compound.

Moreover, the present invention extends to a method for treating, ameliorating or preventing macular degeneration in a patient, comprising administering to the patient an effective amount of a compound that modulates the patient's immune system, wherein the immune system has type I immune responses and type II immune responses, such that administering the compound to the patient increases the type I immune responses in the patient as compared to the type I immune responses in the patient prior to administering of the compound.

Numerous terms and phrases used throughout the instant specification and appended claims are defined below.

As used herein, the term "immune system" refers to a system in a vertebrate that involves direct or indirect action of immunocytes that causes physiological consequences such as inflammation, for example.

As used herein, the term "type I immune responses" refers to immune responses that preferentially activate Th1 and Tc1 responses of the T lymphocytes, and classical activation of macrophages known as M1 activation that is characterized as release of pro-inflammatory mediators, as well as skewing immune responses toward more cell mediated immune response in contrast to the humoral immune responses.

As used herein, the term "type II immune responses" refers to immune responses that preferentially activate Th2 and Tc2 responses of the T lymphocytes and alternative activation of macrophages known as M2 activation that are pro-angiogenic, wound healing or other repair oriented consequences as well as generation of antibodies

As used herein, the phrase "increases the type I responses as compared to the type I responses in the patient prior to administering the compound" refers to increasing immune responses and mediator releases that are pro-inflammatory such as IFNγ, IL-12, IL-23, TNFα, IL-1β, CCL9, CCL10, CCL11 and reduction of mediator release such as IL-10, CCL19, CCL21.

As used herein, the term "immunocyte" refers to cells that are part of the immune system and originate from hematopoietic lineage. These cells contribute to type I immune responses.

As used herein, the term "syk multi-kinase inhibitor" refers to a compound that exhibits a therapeutic effect by simultaneously inhibiting syk kinase as well as several other kinases; not specifically inhibiting only one kinase as a molecular target, but primarily inhibiting syk kinase along with other kinases.

As used herein, the term "hPGDS inhibitor" refers to a compound that inhibits the function of hemotopoietic prostaglandin D2 synthase. This synthase catalyzes the conversion of PGH2 to PGD₂, which in turn is released from mast cells believed to be a mediator of allergic and inflammatory responses

As used herein, the term "DP antagonist" refers to a compound that inhibits the function of the receptor for prostaglandin D₂ , DP1 or DP.

As used herein, the term "choroidal neovascularization" refers to incomplete new vasculature formation initiated from the choroidal compartment that outgrows into the subretinal spaces between Bruch's membrane and retinal pigmented epithelial (RPE) cells.

As used herein, the term "effective amount" refers to an amount sufficient to reduce by at least about 15 percent, preferably by at least 50 percent, more preferably by at least 90 percent, and most preferably prevent, a clinically significant deficit in symptoms or signs of macular degeneration in the host. Alternatively, an effective amount is sufficient to cause an improvement in a clinically significant condition, i.e. macular degeneration, in patient.

The terms "a" and "an" as used herein refer to one or more than one.

As used herein, "patient" refers to the subject being treating according to a method of the present invention. Naturally, a patient can be human, a primate, canine, feline, bovine, equine, murine, etc.

As explained above, the present invention involves a method of treating, preventing or ameliorating macular degeneration, wherein a compound is administered to a patient suffering from macular degeneration. In a particular embodiment, the compound is a component of a pharmaceutical composition, which is administered to the patient. Such pharmaceutical compositions may be for administration for injection, or for oral, pulmonary, nasal or other forms of administration. In general, comprehended by the present invention are pharmaceutical compositions comprising an effective amount of a compound having applications in a method of the present invention with pharmaceutically acceptable diluents, preservatives, solubilizers, emulsifiers, adjuvants and/or carriers. Such compositions include diluents of various buffer content (*e.g*., Tris-HCI, acetate, phosphate), pH and ionic strength; additives such as detergents and solubilizing agents (*e.g*., Tween 80, Polysorbate 80), anti-oxidants (*e.g*., ascorbic acid, sodium metabisulfite), preservatives (*e.g*., Thimersol, benzyl alcohol) and bulking substances (*e.g*., lactose, mannitol); incorporation of the material into particulate preparations of polymeric compounds such as polylactic acid, polyglycolic acid, etc. or into liposomes. Hylauronic acid may also be used. Such compositions may influence the physical state, stability, rate of *in vivo* release, and rate of *in vivo* clearance of a compound having applications in a method of the present invention. *See, e.g*., Remington's Pharmaceutical Sciences, 18th Ed. (1990, Mack Publishing Co., Easton, PA 18042) pages 1435-1712 which are herein incorporated by reference. The compositions may be prepared in liquid form, or may be in dried powder, such as lyophilized form.

### Oral Delivery

Contemplated for use herein are oral solid dosage forms, which are described generally in Remington's Pharmaceutical Sciences, 18th Ed.1990 (Mack Publishing Co. Easton PA 18042) at Chapter 89, which is herein incorporated by reference. Solid dosage forms include tablets, capsules, pills, troches or lozenges, cachets or pellets. Also, liposomal or proteinoid encapsulation may be used to formulate a compound having applications in a method of the present invention (as, for example, proteinoid microspheres reported in U.S. Patent No. 4,925,673). Liposomal encapsulation may be used and the liposomes may be derivatized with various polymers (*e.g*., U. S. Patent No. 5,013,556). A description of possible solid dosage forms for the therapeutic is given by Marshall, K. In: Modern Pharmaceutics Edited by G. S. Banker and C.T. Rhodes Chapter 10, 1979. In general, the formulation will include a compound having applications in a method of the present invention and inert ingredients which allow for protection against the stomach environment, and release of the compound in the intestine.

Also specifically contemplated are oral dosage forms of a compound having applications in a method of the present invention. Moreover, it is desirable that the overall stability of the dosage form is increased in order to increase the circulation time of the compound in the body.

The location of release may be the stomach, the small intestine (the duodenum, the jejunum, or the ileum), or the large intestine. One skilled in the art has available formulations which will not dissolve in the stomach, yet will release a compound having applications in a method of the present invention in the duodenum or elsewhere in the intestine. Preferably, the release will avoid the deleterious effects of the stomach environment, either by protection of the protein or by release of the compound beyond the stomach environment, such as in the intestine.

To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), EUDRAGIT L30D, AQUATERIC, cellulose acetate phthalate (CAP), EUDRAGIT L, EUDRAGIT S, and shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic i.e. powder; for liquid forms, a soft gelatin shell may be used. The shell material of cachets could be thick starch or other edible paper. For pills, lozenges, molded tablets or tablet triturates, moist massing techniques can be used.

A compound having applications in a method of the present invention can also be included in a formulation as fine multi-particulates in the form of granules or pellets of particle size about 1 mm. The formulation of the compound for capsule administration could also be as a powder, lightly compressed plugs or even as tablets. The therapeutic could be prepared by compression. Moreover, the compound can be in a crystalline or amorphous form.

Colorants and flavoring agents may all be included. For example, the compound may be formulated (such as by liposome or microsphere encapsulation) and then further contained within an edible product, such as a refrigerated beverage containing colorants and flavoring agents.

One may dilute or increase the volume of the compound with an inert material. These diluents could include carbohydrates, especially mannitol, α-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of a compound having applications in a method of the present invention into a solid dosage form. Materials used as disintegrates include but are not limited to starch, including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold a pharmaceutical composition containing a compound having applications in a method of the present invention together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An anti-frictional agent may be included in a formulation of a compound having applications in a method of the present invention to prevent sticking during the formulation process. Lubricants may be used as a layer between the compound and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the compound during formulation and to aid rearrangement during compression might be added. Such glidants may include starch, talc, pyrogenic silica and hydrated silicoaluminate, to name only a few.

To aid dissolution of a compound having applications in a method of the present invention into an aqueous environment, a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. The list of potential non-ionic detergents that could be included in a formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in a formulation of a compound having applications in a method of the present invention either alone or as a mixture of such compounds.

Additives which potentially enhance uptake of a compound having applications in a method of the present invention are for instance the fatty acids oleic acid, linoleic acid and linolenic acid.

Controlled release oral formulation may be desirable. A compound having applications in a method of the present invention could be incorporated into an inert matrix which permits release by either diffusion or leaching mechanisms, *e.g*., gums. Slowly degenerating matrices may also be incorporated into the formulation. Some enteric coatings also have a delayed release effect.

Another form of a controlled release of a compound having applications in a method of the present invention is by a method based on the Oros therapeutic system (Alza Corp.), i.e. the compound is enclosed in a semipermeable membrane which allows water to enter and push the compound out through a single small opening due to osmotic effects.

Other coatings may be used for the formulation. These include a variety of sugars which could be applied in a coating pan. A compound having applications in a method of the present invention could also be given in a film coated tablet and the materials used in this instance are divided into 2 groups. The first are the nonenteric materials and include methyl cellulose, ethyl cellulose, hydroxyethyl cellulose, methylhydroxy-ethyl cellulose, hydroxypropyl cellulose, hydroxypropyl-methyl cellulose, sodium carboxy-methyl cellulose, providone and the polyethylene glycols. The second group consists of the enteric materials that are commonly esters of phthalic acid.

A mix of materials might be used to provide the optimum film coating. Film coating may be carried out in a pan-coater or in a fluidized bed or by compression coating.

*Pulmonary Delivery.* Also contemplated herein is pulmonary delivery of a compound having applications in a method of the present invention. The compound is delivered to the lungs of a mammal while inhaling and traverses across the lung epithelial lining to the blood stream. Other reports of this include Adjei et al., 1990, Pharmaceutical Research, 7:565-569; Adjei et al., 1990, International Journal of Pharmaceutics, 63:135-144 (leuprolide acetate); Braquet et al., 1989, Journal of Cardiovascular Pharmacology, 13(suppl. 5):143-146 (endothelin-1); Hubbard et al., 1989, Annals of Internal Medicine, Vol. III, pp. 206-212 (al- antitrypsin); Smith et al., 1989, J. Clin. Invest. 84:1145-1146 (a-1-proteinase); Oswein et al., 1990, "Aerosolization of Proteins", Proceedings of Symposium on Respiratory Drug Delivery II, Keystone, Colorado, March, (recombinant human growth hormone); Debs et al., 1988, J. Immunol. 140:3482-3488 (interferon-g and tumor necrosis factor alpha) and Platz et al., U.S. Patent No. 5,284,656 (granulocyte colony stimulating factor). A method and composition for pulmonary delivery of drugs for systemic effect is described in U.S. Patent No. 5,451,569, issued September 19, 1995 to Wong et al.

Contemplated for use in the practice of a method of the present invention are a wide range of mechanical devices designed for pulmonary delivery of a compound having applications in a method of the present invention, including but not limited to nebulizers, metered dose inhalers, and powder inhalers, all of which are familiar to those skilled in the art.

Some specific examples of commercially available devices suitable for the practice of this invention are the ULTRAVENT nebulizer, manufactured by Mallinckrodt, Inc., St. Louis, Missouri; the ACORN II nebulizer, manufactured by Marquest Medical Products, Englewood, Colorado; the VENTOLIN metered dose inhaler, manufactured by Glaxo Inc., Research Triangle Park, North Carolina; and the SPINHALER powder inhaler, manufactured by Fisons Corp., Bedford, Massachusetts, and the ULTRAHALER manufactured by sanofi-aventis, to name only a few.

All such devices require the use of formulations suitable for the dispensing of a compound having applications in a method of the present invention. Typically, each formulation is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated.

Formulations suitable for use with a nebulizer, either jet or ultrasonic, will typically comprise a compound having applications in a method of the present invention dissolved in water at a concentration of about 0.1 to 25 mg of compound per mL of solution. The formulation may also include a buffer and a simple sugar (*e.g*., for stabilization and regulation of osmotic pressure). The nebulizer formulation may also contain a surfactant to reduce or prevent surface induced aggregation of the compound caused by atomization of the solution in forming the aerosol.

Formulations for use with a metered-dose inhaler device will generally comprise a finely divided powder containing a compound having applications in a method of the present invention, suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Other propellants having applications herein are hydrofluoroalkanes, which have a general formula of CₓH_{y}F_{z}, in which x is an integer from 1 to 3, y+z =2x+2 and y and z are both at least 1. Particular hydrofluoroalkanes having applications herein are CF₃CFH₂, CH₃CHF₂ and CF₃CHFCF₃. In general, the vapor pressure of the propellant mixture should be in the range suitable and permitted for aerosol propellants. The vapor pressure may be varied by mixing one or more hydrofluoroalkanes and/or some other suitable vapor pressure modifying agent in appropriate proportions

Suitable surfactants include sorbitan trioleate and soya lecithin. Oleic acid may also be useful as a surfactant. Moreover, the compound can be in either crystalline or amorphous form.

Formulations for dispensing from a powder inhaler device will comprise a finely divided dry powder of a compound having applications in a method of the present invention, and may also include a bulking agent, such as lactose, sorbitol, sucrose, or mannitol in amounts which facilitate dispersal of the powder from the device, *e.g*., 50 to 90% by weight of the formulation. The compound should most advantageously be prepared in particulate form with an average particle size of less than 10 mm (or microns), most preferably 0.5 to 5 mm, for most effective delivery to the distal lung.

*Nasal Delivery.* Nasal delivery of a compound having applications in a method of the present invention is also contemplated. Nasal delivery allows the passage of the compound to the blood stream directly after administering the therapeutic product to the nose, without the necessity for deposition of the product in the lung. Formulations for nasal delivery include those with dextran or cyclodextran.

*Transdermal delivery.* Various and numerous methods are known in the art for transdermal administration of a drug, *e.g*., via a transdermal patch. Transdermal patches are described in for example, U. S. Patent No. 5,407,713, issued April 18, 1995 to Rolando et al.; U.S. Patent No. 5,352,456, issued October 4, 1004 to Fallon et al.; U.S. Patent No. 5,332,213 issued August 9, 1994 to D'Angelo et al.; U.S. Patent No. 5,336,168, issued August 9, 1994 to Sibalis; U.S. Patent No. 5,290,561, issued March 1, 1994 to Farhadieh et al.; U.S. Patent No. 5,254,346, issued October 19, 1993 to Tucker et al.; U.S. Patent No. 5,164,189, issued November 17, 1992 to Berger et al.; U.S. Patent No. 5,163,899, issued November 17, 1992 to Sibalis; U.S. Patent Nos. 5,088,977 and 5,087,240, both issued February 18, 1992 to Sibalis; U.S. Patent No. 5,008,110, issued April 16, 1991 to Benecke et al.; and U.S. Patent No. 4,921,475, issued May 1, 1990 to Sibalis.

It can be readily appreciated that a transdermal route of administration may be enhanced by use of a dermal penetration enhancer, *e.g*., such as enhancers described in U.S. Patent No. 5,164,189 (*supra*), U.S. Patent No. 5,008,110 (*supra*), and U.S. Patent No. 4,879,119, issued November 7, 1989 to Aruga et al., the disclosure of each of which is incorporated herein by reference in its entirety.

### Topical Eve Delivery

Compositions suitable for topical administration are known to the art (see, for example, U.S. Patent Application Publication No. 2005/0059639). In various embodiments, compositions of a compound having applications in a method of the present invention can comprise a liquid comprising a compound in solution, in suspension, or both. As used herein, liquid compositions include gels. Preferably the liquid composition is aqueous. Alternatively, the composition can take form of an ointment. In a preferred embodiment, the composition is an *in situ* gellable aqueous composition, more preferably an *in situ* gellable aqueous solution. Such a composition can comprise a gelling agent in a concentration effective to promote gelling upon contact with the eye or lacrimal fluid in the exterior of the eye. Aqueous compositions of the invention have ophthalmically compatible pH and osmolality.

Topical application (dropped into the eye) of a compound having applications in a method of the present invention may permit one to avoid most of the systemic side effects, but for effectiveness it is essential that the compound or compounds be actively transported across the cornea at sufficient levels to reach the proper receptor site.

Typically, administration of a compound having applications in a method of the present invention involves preparing an ointment (such as petrolatum) or gel with a conventional pharmaceutical carrier (such as carbopol) and topically administering the gel composition. The amount of the compound in the composition should be from about 0.25% by weight to about 5% by weight for an eye drop composition, preferably from about 0.5% by weight to about 2.0% by weight. The important point is not the dose amount, but simply that it be an amount that is effective in treating, preventing or ameliorating macular degeneration and yet not be so strong as to provide eye irritation or side effects. Generally, amounts within the ranges herein specified are satisfactory. Moreover, one of ordinary skill in the art can readily determine the appropriate ranges using routine laboratory techniques.

The carrier for the eye drop composition may be an isotonic eye treatment carrier buffered to a pH of from about 4 to about 8, and typically it will contain small amounts of conventional wetting agents and anti-bacterial agents. The preferred pH is within the range of from about 6.8 to about 7.8 and contains sufficient sodium chloride or equivalent to be isotonic. Anti-bacterial agents where they are included may be within the range of from about 0.004% (W/V) to about 0.02% (W/V) of the composition.

A compound having applications in a method of the present invention may be incorporated into various ophthalmic gel formulations for delivery to the eye. In order to form sterile ophthalmic ointment formulations, the compound may be combined with a preservative in an appropriate vehicle, such as mineral oil, liquid lanolin, or white petrolatum. Sterile ophthalmic gel formulations may be prepared by suspending a compound having applications in a method of the present invention in a hydrophilic base prepared from a combination of carbopol -940 (a carboxy vinyl polymer available from the B. F. Goodrich Company) according to published formulations for analogous ophthalmic preparations. Preservatives and tonicity agents can also be incorporated.

### Subtenon Delivery

Subtenon delivery of a compound having applications in a method of the present invention also can be used to treat, prevent or ameliorate macular degeneration. A particular example of such delivery is set forth in US Patent 6,413,245, which discloses a method and apparatus for delivering a drug formulation to a human eye. The method includes the steps of inserting the apparatus below the Tenon's capsule and above the sclera at a point posterior to the limbus of the eye and injecting the drug formulation to form a drug depot on an outer surface of the sclera. The apparatus includes a cannula having a distal portion, a proximal portion, and a bend separating the distal portion and the proximal portion. The distal portion has a radius of curvature substantially equal to a radius of curvature of the globe of the eye. A tangent of the distal portion at the bend is disposed at an angle no more than about 56 degrees with respect to the proximal portion. Other subtenon delivery methods and apparati known to one of ordinary skill in this art readily applications herein.

### Intravitreal Delivery

The composition can comprise an ophthalmic depot formulation comprising a compound having applications in a method of the present invention for subconjunctival administration. The microparticles comprising the compound can be embedded in a biocompatible pharmaceutically acceptable polymer or a lipid encapsulating agent. The depot formulations may be adapted to release all or substantially all the compound over an extended period of time. The polymer or lipid matrix, if present, may be adapted to degrade sufficiently to be transported from the site of administration after release of all or substantially all the compound. The depot formulation can be a liquid formulation, comprising a pharmaceutical acceptable polymer and a dissolved or dispersed active agent. Upon injection, the polymer forms a depot at the injections site, e.g. by gelifying or precipitating. The composition can comprise a solid article that can be inserted in a suitable location in the eye, such as between the eye and eyelid or in the conjuctival sac, where the article releases the compound. Solid articles suitable for implantation in the eye in such fashion generally comprise polymers and can be bioerodible or non-bioerodible.

### Methods of Treatment, Methods of Preparing a Medicament

As explained above, methods for treating, preventing or ameliorating macular degeneration are provided.

*Dosages.* In a method of the present invention, as further studies are conducted, information will emerge regarding appropriate dosage levels for prevention, treatment or amelioration of macular degeneration in various patients, and the ordinary skilled worker, considering the therapeutic context, age and general health of the patient, will be able to ascertain proper dosing using merely routine laboratory techniques.

*Administration with other compounds.* Naturally, a compound having applications in a method of the present invention to treat, prevent or ameliorate a macular degeneration may be administered in conjunction with one or more pharmaceutical compositions used for treating other clinical symptoms, such as choroidal angiogenesis, which may be related to the macular degeneration. Anti-angigenic pharmaceuticals for the treatment of wet age-related macular degeneration such as LUCENTIS and AVASTIN can be used as a combination with a compound having applications in a method of the present invention so that intravitreal injection interval of LUCENTIS or AVASTIN can be lengthened. The present invention may be better understood by reference to the following non-limiting Examples, which are provided as exemplary of the invention. The following examples are presented in order to more fully illustrate the preferred embodiments of the invention. They should in no way be construed, however, as limiting the broad scope of the invention.

### EXAMPLE 1

Fifty male Brown Norway (strain BN/RijHsd) rats (Rattus norvegicus), 4-7 weeks old (50-124 g each), were purchased from Harlan (Indianapolis, IN), received in good condition. The animals were quarantined and acclimated for three (3) days. During the quarantine period, the animals were observed at least once daily for clinical signs of abnormality. 50 animals were arbitrarily assigned to study groups and given permanent identification numbers.

Upon assignment to a study group, each animal was weighed and tail-marked with a unique permanent identification number using indelible ink. A cage card displaying the animals' permanent identification numbers and study number was displayed throughout the in-life period.

### Animal husbandry

Controls were set to maintain the temperature and relative humidity of the animal rooms at 64-79 °F and 30-70%, respectively. These environmental parameters were monitored and recorded daily. Twelve hours of light and twelve hours of dark were provided in the animal rooms. A fluorescent light source was used, with lights turned on at approximately 0700 hours and turned off at approximately 1900 hours each day. LABDIET® 5001 Rodent Diet (Purina Mills, Inc., St. Louis, MO) was fed ad libitum throughout the acclimation and in-life periods. Records of lot number(s) and certificates of analysis are maintained by the Testing Facility. There are no known contaminants that are reasonably expected to be present in the diet that are known to be capable of interfering with the purpose or conduct of the study. Fresh water from the Municipal Water Supply was provided ad libitum to the animals via a rack watering system. The water supply is periodically monitored for chlorine content and bacterial contamination. Animals were housed individually or in compatible pairs in plastic "shoe-box" cages within a room dedicated to rodents. General procedures for animal housing and husbandry were conducted according to Testing Facility SOPs and met all regulations concerning use of animals in research, including the U.S. Department of Agriculture regulations (9 CFR Chapter 1) implementing the Animal Welfare Act (21 USC 2131 et seq.) and the recommendations of the National Research Council's Guide for Care and Use of Laboratory Animals (National Academy Press, 1996).

### Laser Surgery

The study was conducted in accordance with a research proposal approved by the Institutional Animal Care and Use Committee.

The retinae (both eyes of each rat) were lased using a retinal lasering device with an attached Kowa PortSlit, SC14; Keeler Fison Indirect Ophthalmoscope (Lens 30 Diopter). Rats were briefly anesthetized and the eyes were dilated and coverslips were used as necessary to flatten the cornea. Each retina was lased with 186mW, 75 µm spot sizes, 0.1 sec duration, applied to (approximately) 9-, 12- and 3-o'clock positions, 2-3 disk diameters from the optic nerve of each eye as feasible as determined by the surgeon. Rupture of the Bruch's membrane was identified by bubble formation, which occurred immediately at the site of photocoagulation. Rats were briefly anesthetized and administered fluoroscein to visualize the retinal vasculature post surgically and prior to necropsy. Representative photographs of the retinas were taken using a Kowa retinal camera for rodents. The photographs were taken to assure that vascular lesions were present immediately following the laser procedure and immediately prior to necropsy.

### Dose Administration

2-[4-(7-ethyl-5H-pyrrolo[2,3-b]pyrazin-6-yl)-phenyl]-propan-2-ol A and 2-(3-fluoro-phenyl)-4-methyl-pyrimidine-5-carboxylic acid indol-1-ylamide were formulated in vehicle twice weekly during the study. Vehicle consisted of 0.5% methylcellulose, 0.2% Tween 20 in sterile water for injection, and was formulated once weekly. 2-(3-fluoro-phenyl)-4-methyl-pyrimidine-5-carboxylic acid indol-1-ylamide test article was reconstituted in vehicle at 0.6, 2, and 6 mg/mL; 2-(3-fluoro-phenyl)-4-methylpyrimidine-5-carboxylic acid indol-1-ylamide control article was reconstituted at 2 mg/mL. All solutions were stored refrigerated at 2-8 °C between dose administrations, and mixed prior to each dose administration.

Animals were dosed via twice daily oral gavage at 5 mL/kg, starting immediately prior to lasing and continuing for 14 days. Dose volumes were recalculated weekly based on the most recent body weight. Note that animals were lased on two sequential days, but were sacrificed together on a single day to facilitate handling during necropsy. Therefore, animals of the first cohort (Groups 1-3) received an extra (29th) dose on the morning of Day 14 to permit terminal bleed and sacrifice within 2-4 hr of final dose administration.

### Clinical Observations

Clinical observations, including morbidity, mortality, and overt signs of toxic or pharmacologic effect(s) were recorded daily for individual animals throughout the in-life periods (quarantine/acclimation and treatment). Any and all signs of clinical abnormality were recorded.

### Body Weights

Body weights were recorded prior to dosing, at one week, and at necropsy.

### Fundus Photography

The fundus was photographed on Day 0, at the time of lasing or shortly thereafter, and on Day 13 or 14, prior to sacrifice. At each time point, retinal vasculature was illuminated by intravenous (IV) injection of each animal, several minutes before photography, with 0.1 mL of a 10% solution of fluorescein dye. Each eye was dilated with a mydriatic and photographed with Kowa Small Animal Fundus Camera. The purpose of the fundus photography was to confirm the presence lesions following lasing and prior to necropsy. Any animal found not to have lesions would be removed from study and replaced.

### Necropsy

Following fundus photography and terminal bleeds, animals were sacrificed per SOPs. From each animal, the right eye was collected into 10% NBF, and the left eye was collected into a frozen OCT block.

### Histopathology

Following processing of the NBF-fixed right eyes for histopathology, sectioning was performed as follows: each fixed eye was oriented sagittally and then 10-15 step sections were cut through the central part of the eye including the retinal-optic nerve region. Since 4-6 sections can be placed on one slide, there were typically 4-5 slides per eye. At least one lesion and preferably 2 or more lesions were sought.

The slides were evaluated visually, qualitatively and semi-quantitatively. The nuclei of new vessels growing on the retinal were counted. Evaluation typically included counting of ∼6 high-powered fields from 4-6 representative retinal sections. The data was entered into a spreadsheet and the means and standard deviations were calculated using Excel® (Office 2000; Microsoft, Redmond, WA).

The remaining (left) eyes were stored frozen pending future immunohistochemical evaluation of immunocyte infiltration.

### Statistical Analysis

Body weights were analyzed using the One-Way Analysis of Variance (ANOVA) function of EXCEL (OFFICE 2000; Microsoft, Redmond, WA). P values of ≤0.05 were considered statistically significant.

### EXAMPLE 2

### Test System

Thirty-five male and thirty-five female Brown Norway rats (Rattus norvegicus; strain BN/MCW), 6-7 weeks old, were purchased from Charles River Laboratories (Portage, MI). The animals were acclimated for seven (7) days. During the acclimation period, the animals were observed at least once daily for clinical signs of abnormality, the animals appeared clinically normal and were released for use in the study. All seventy animals were weighed, and thirty males and thirty females were randomly assigned to study groups and given permanent identification numbers. Upon assignment to a study group, each animal was weighed and tail-marked with a unique permanent identification number using indelible ink. A cage card displaying the animals' permanent identification numbers and study number was displayed throughout the in-life period.

### Animal husbandry

Controls were set to maintain the temperature and relative humidity of the animal rooms at 64°-79° F and 30-70%, respectively. These environmental parameters were monitored and recorded daily. Twelve hours of light and twelve hours of dark were provided in the animal rooms. A fluorescent light source was used, with lights turned on at approximately 0700 hours and turned off at approximately 1900 hours each day. LABDIET 5001 Rodent Diet (Purina Mills, Inc., St. Louis, MO) was fed ad libitum throughout the acclimation and in-life periods. Records of lot number(s) and certificates of analysis are maintained by the Testing Facility. There are no known contaminants that are reasonably expected to be present in the diet that are known to be capable of interfering with the purpose or conduct of the study. Fresh water from the Municipal Water Supply was provided ad libitum to the animals via a rack watering system. The water supply is periodically monitored for chlorine content and bacterial contamination. Animals were housed individually or in compatible pairs in plastic "shoe-box" cages within a room dedicated to rodents. General procedures for animal housing and husbandry were conducted according to Testing Facility SOPs and met all regulations concerning use of animals in research, including the U.S. Department of Agriculture regulations (9 CFR Chapter 1) implementing the Animal Welfare Act (21 USC 2131 et seq.) and the recommendations of the National Research Council's Guide for Care and Use of Laboratory Animals (National Academy Press, 1996).

### Laser Surgery and Ophthalmology

The study was conducted in accordance with a research proposal approved by the Institutional Animal Care and Use Committee.

Laser surgery was performed on the retinae of both eyes using a modified slit lamp laser delivery system. An IRIDEX Diovet diode laser was used, delivering 810 nm wave length of light. The laser delivered 170 mW to a 75 um spot for 0.075 seconds. Three laser lesions were targeted at approximately 9, 12 and 3 o'clock positions, 2-3 disk-diameters from the optic nerve of each eye. Laser conditions were optimized to produce acute vapor bubbles at Bruch's membrane, indicative of membrane rupture. Coverslips were used by the surgeon as necessary to flatten the cornea to aid her in targeting/aiming the laser.

Immediately prior to surgery (Day 1) and prior to necropsy (Day 13), rats were briefly anesthetized by intramuscular injection with 0.15 mL/rat of a ketamine/xylazine cocktail. This dose level corresponded to a dose of ∼60 mg/kg ketamine and ∼8 mg/kg xylazine, furnishing about 30 min of anesthesia for lasering and post surgical photography (Day 1), and for terminal photography (at necropsy). On both days, fluorescein (0.1 mL of 10% solution) was administered intravenously immediately prior to photography, permitting visualization of the retinal vasculature. Representative photographs of the retinae were taken using a Kowa Small Animal Fundus Camera. The photographs were taken to ensure that vascular lesions were present immediately following the laser procedure and immediately prior to necropsy to assess the lesion size 14-days after laser surgery. If any animals did not display post laser lesions on Day 1, they would have been removed from study and replaced. In this study, there were no replacements for this reason.

### Compounds preparation

2-[4-(7-ethyl-5H-pyrrolo[2,3-b]pyrazin-6-yl)-phenyl]-propan-2-ol, phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt, and_2-(3-{6-[2-(2,4-dichloro-phenyl)-ethylamino]-2-methoxy-pyrimidin-4-yl}-phenyl)-2-methyl-propionic acid phosphoric acid salt were each resuspended separately in the vehicle consisting of 0.5% methylcellulose, 0.2% Tween 20 in sterile water. The vehicle was formulated from components as follows: methylcellulose: Spectrum Chemical Mfg. Corp. Catalog ME136 (Lot NI0534; exp. December 2008); Tween 20: Sigma-Aldrich (St. Louis, MO) Catalog X251-07 (Lot X22H09; exp. December 2010); and Sterile Water for Injection, USP. The components were stored at controlled room temperature; following formulation (once, at the start of the study), vehicle was stored refrigerated at 2-8 °C.

### Dose Administration

A single batch of vehicle (0.5% methylcellulose, 0.2% Tween 20 in sterile water) was prepared on Day 0 and used for all subsequent formulations of test articles. Vehicle was stored refrigerated at 2-8 °C throughout the study. Animals were dosed twice daily, mid-morning and mid-afternoon, with about six hours between dose administrations on each study day.

### Clinical Observations

Clinical observations, including morbidity, mortality, and overt signs of toxic or pharmacologic effect(s) were recorded at least once daily for individual animals throughout the in-life periods (quarantine/acclimation and treatment). Any and all signs of clinical abnormality were recorded.

### Body Weights

Body weights were recorded prior to dosing, at one week, and at necropsy.

### Necropsy

Animals that died during the study were subjected to gross necropsies to determine possible causes of death. The remaining (surviving) animals were sacrificed on Day 13 following fundus photography and terminal blood collection. From each animal, the right eye was collected into modified Davidson's solution (for histopathological evaluation), and the left eye was frozen in OCT embedding compound [for possible immunohistochemistry (IHC)].

### Histopathology

Following processing of the modified Davidson's-fixed right eyes for histopathology, sectioning was performed as follows: each fixed eye was oriented sagittally and then 10-15 step sections were cut through the central part of the eye, including the retinal-optic nerve region. Since 4-6 sections can be placed on one slide, there were typically 4-5 slides per eye, enabling the pathologist to view at least two lesions per animal.

The lesions were measured with a reticle eye piece in a horizontal and lateral orientation at 400x magnification. Three sections were selected for measurement. The lesion estimated to be closest to the laser lesion site and the section immediately anterior and posterior to this site were selected for measurement. The data were recorded onto an Excel spreadsheet. The mean width and length with group means and standards deviation were calculated for each group. The area of each lesion was calculated as a triangle for each site in each animal. Since measurements were made above and below the primary lesion, a volume estimate was also calculated.

### Statistical Analysis

Body weights were analyzed using the One-Way Analysis of Variance (ANOVA) function of EXCEL (OFFICE 2000; Microsoft, Redmond, WA). P values of ≤0.05 were considered statistically significant.

### Example 3

In this example, the effect of DP antagonist 2-(3-{6-[2-(2,4-dichloro-phenyl)-ethylamino]-2-methoxy-pyrimidin-4-yl}-phenyl)-2-methyl-propionic acid phosphoric acid salt (the compound) on plaque volume of choroidal neovascularization in the rat laser-induced macular degeneration model. As in the Examples discussed above, brown Norway rats (Rattus norvegicus) were used in this example. The rats received laser coagulation on day 0. , 4-7 weeks old (50-124 g each), were purchased from Harlan (Indianapolis, IN), received in good condition. The animals were quarantined and acclimated for three (3) days. The compound was then administered orally twice each day on days -1 to 14. Indomethacin (positive control) was similarly administered twice daily. On day 14, the rats were sacrificed and choroidal neovascular (CNV) volumes were measured on day 14.

The histogram of FIG. 4 shows that the compound administered in a model that mimics maculular degeneration was very effective in limiting the volume of choroidal neovascularization.

### Example 4

In this example, the same experiment was performed with the rat laser-induced macular degeneration model to determine whether the hPDGS inhibitor 4-Methyl-2-pyridin-2-yl-pyrimidine-5-carboxylic acid (5-fluoro-3-methyl-indol-1-yl)-amide could limit the plaque volume of choroidal neovascularization

In particular, the hPDGS inhibitor 4-Methyl-2-pyridin-2-yl-pyrimidine-5-carboxylic acid (5-fluoro-3-methyl-indol-1-yl)-amide (the compound) was administered orally to the rats twice each day on days - 1 to day 14. On day 0, a laser was used to induce choroidal neovascularization in the eye of the brown Norway rats. Indomethacin was administered to a control group similarly, i.e., twice each day from day -1 to day 14.

After fourteen days, the rats were sacrificed and the plaque volume of choroidal neovascularization in the eye was measured. The results, which are set forth in FIG. 5, show that the compound was indeed effective in limiting the plaque volume of choroidal neovascularization in the rats tested, compared with the control.

The histogram of FIG. 5 shows that the compound administered in a model that mimics maculular degeneration was very effective in limiting the plaque volume of choroidal neovascularization.

### Example 5

In this example, the effect of hPGDS antagonist 2-Pyridin-2-yl-pyrimidine-5-carboxylic acid 3-[5-(1-hydroxy-1-methyl-ethyl)-1,2,4-oxadiazol-3-yl]-benzylamide was evaluated using the rat laser-induced macular degeneration model. In particular, 2-Pyridin-2-yl-pyrimidine-5-carboxylic acid 3-[5-(1-hydroxy-1-methyl-ethyl)-1,2,4-oxadiazol-3-yl]-benzylamide was administered orally to the rats twice each day on days -1 to 7. Doxycycline, a positive control, was orally administered once daily. Choroidal neovascular (CNV) volumes were measured on day 7.

### Test Article

The compound was stored at controlled room temperature. The compound was suspended in vehicle, 0.5% methyl cellulose 0.2% tween 80.

### Vehicle and Positive Control Article

The negative control article was the vehicle for the test articles, and consisted of 0.5% methyl cellulose, 0.2% Tween 80 in distilled water. The vehicle was formulated from components as follows: methyl cellulose, Sigma Chemical. (St Louis, MO) Catalog # M-0262 (Lot 017k0087); Tween 80, Acros Organics (Geel, Belgium) Catalog # 278632500 (Lot A0265286); and deionized water (in house), The components were stored at controlled room temperature; following formulation, vehicle was refrigerated at 2-8°C. The test article was prepared by suspending the test material in vehicle using Retsch mixer mill model MM301 Retsch Industries (Newtown, PA) shaken for 10 minutes at 15 oscillations/second with 2.0 mm yttria stab zir oxide beads, Fox industries (Fairfield NJ), Lot #12974. The Test article was stored in glass bottles The control article was Sigma-Aldrich doxycycline (St Louis, MO) Catalog # D9891, Lot 18K0709). Doxycycline ( 3 mg/ml) was dissolved in the same vehicle as the test articles (0.5% methyl cellulose, 0.2% tween 80). Following formulation all test materials were stored in glass bottles and protected from light and stored refrigerated at 2-8 °C when not being dosed. Doxycycline was stored in a brown glass bottle.

### Test System

Sixty (60) male BN rats (*Rattus norvegicus*; strain Brown Norway) approximately 8 weeks old, were purchased from Harlan (Livermore, CA).

### Use of animals

This study is in accordance with the sanofi-aventis group policy on the use of animals in research.

### Receipt and Acclimation

Sixty animals each were received in good condition. The animals were acclimated for a minimum of 5 days. During the acclimation period, the animals were observed for any clinical signs of abnormality. All animals were in good condition.

### Dosing Cohorts

Animals were dosed and underwent laser coagulation as two separate cohorts.

### Identification

The animals were selected randomly and each animal was tail-marked with a unique permanent identification number using indelible ink. A cage card displaying the animals' permanent identification numbers and study number was displayed throughout the in-life period.

### Animal Housing Conditions

Animals were housed under conditions outlined in the NIH Guide for Care and Use of Laboratory Animals in compliance with the USDA Laboratory Animal Welfare act, in a fully accredited AAALAC facility. They were housed 3 per cage. Twelve hours of light and twelve hours of dark were provided in the animal rooms. Animals were fed Harlan Global Teklad Diet 2016 and filtered water *ad libitum*.

### Final Selection of Animals

The first half of animals (n=30) (Cohort A) received laser coagulation on one day and the other half (n=30) (Cohort B) received laser coagulation on the second day. Studies began with twelve animals per group in case of the need for replacement of an animal due to accidental death during lasering or poor laser delivery (i.e. accidental retinal vessel hit and bleeding). No accidental deaths occurred. If no errors occurred during lasering for a particular group, rats were randomly removed.

### Laser Coagulation

On the morning of Day 0, the day after the first dose administration, animals were transported to the laser delivery procedure room. Laser surgery was performed on the retina of the left eye only of each animal using a modified slit lamp laser delivery system.

### The surgical procedure was as follows.

Rats were anesthetized via intraperitoneal (IP) injection of ketamine/medetomidine at 40/0.25 mg/kg (per Testing Facility SOP). The pupil of each eye was dilated by administration of topical (ocular) 1% tropicamide (MYDRACIL, Alcon Labs).

Animals were placed before a modified slit-lamp laser delivery system. The fundus was visualized using a VOLK contact lens set on the system. The fundus of the left eye was treated at 3 sites, at approximately 12-, 3-, and 9-o'clock positions, 2 disk-diameters from the optic nerve of each eye to the best of the ability of the surgeon. This was done using a green argon laser (Coherent Novus 2000; 514-nm wavelength) with a modified slit lamp delivery system (Carl Zeiss Slit Lamp). Each treatment site (200 mW delivered at 0.5 sec pulse duration with 100 µm spot size) was optimized to produce acute vapor bubbles beneath the retina, indicative of rupture of Bruch's membrane.

Following surgery, animals received an intramuscular (IM) injection of ANTISEDAN at 0.25 mg/kg for anesthesia reversal. A drop of GENTEAL (Novartis) eye ointment was placed onto the lasered eye for dry eye relief. Animals were monitored until they fully recovered from anesthesia and were placed back into their animal room.

### Dose Administration

Two batches of vehicle (0.5% methylcellulose, 0.2% Tween 80) were prepared prior to the study and used for all subsequent formulations of test articles. Vehicle was refrigerated at 2-8 °C throughout the study. Two batches of test articles were prepared. One batch was prepared on day -1 and used to dose animals from day -1 to day 3 and the second batch prepared on day 4 was used for all subsequent dosing. Animals were dosed by oral gavage twice daily, early-morning and mid-afternoon, with about 8-9 hours between dose administrations on each study day. Animals were weighed on day -1, 4 and 7 of the study and these weights were used to determine the dosing volume.

### Pharmacokinetic Analysis

On Day 7, rats received the final dose administration during the morning and at specific timepoints, 0.5, 1, and 4 hours (3 animals per group), animals were placed in the CO₂ chamber until breathing cessation and terminal cardiocentesis was performed. Immediately following blood collection, eyes were harvested. The left lasered eye was placed in 10% formalin solution for flat mounting and the right eye was placed into a pre-weighed glass vial and frozen on dry ice. Blood was collected into BD heparinized Microtainer tubes to obtain plasma. The samples were centrifuged and the resulting plasma was frozen on dry ice. Samples were then analyzed.

### Necropsy

Animals of Cohorts A and B were sacrificed on the respective Day +7. From each animal, the lasered left eye right eye was collected into 10% formalin solution for dissection and flat mounting. The right eye was collected and frozen for PK analysis.

### Flat-Mounting, Fluorescence Microscopy and 3D Image Modeling

On the respective Day +7, the left lasered eyes were removed from the formalin solution and processed for flat mounting. The eye was micro-dissected using a dissection microscope. The anterior section and lens was removed to obtain the posterior eye cup. The neural retina was carefully separated from the RPE/choroidal region by gently pulling the tissues away from each other. The RPE/choroid region and the neural retina were "flowered" by making anywhere from 3-6 evenly spaced radial cuts. The tissues were flattened and mounted separately on glass slides with mounting medium (Vectashield HardSet) and coverslip. They were stored at 4°C in the dark until fluorescence analysis.

The lesion volume were measured based on fluorescence (FITC) and 3D modeling using the Zeiss AXIOIMAGEr with APOTOME and AXIOVISION software. All RPE/choroidal slides were analyzed for fluorescence. All analysis was done blindly by the user. For most slides, 2-3 lesions were identified. For few slides, only 1 lesion was identified or none at all. If the tissue showed very low fluorescence (poor perfusion), then the sample was removed from analysis. Laser lesions were identified in the RPE/choroidal flat mounts. Imaging of the laser lesions were done at 20x magnification with an exposure of 150 msec. Images were acquired as Z-stacks using the APOTOME which provides de-blurred optical sections necessary for 3D image analysis. Volume was measured using the AXIOVISION 3D measurement tool and data was exported into Excel.

The results, which are shown in the histograms of FIG. 6A, 6B and 6C show that 2-Pyridin-2-yl-pyrimidine-5-carboxylic acid 3-[5-(1-hydroxy-1-methyl-ethyl)-1,2,4-oxadiazol-3-yl]-benzylamide is indeed effective in limiting CNV volume.

### CONCLUSION

The results of the examples set forth above clearly show that the type of compounds discussed above affect amelioration of the rat laser induced macular degeneration function. These results, in conjunction with references cited above that show PGD2 and DP as well as blocking of mast cell degranulation by Syk multikinase inhibitors alter the activity of immunocytes toward type II immune responses by altering function of mast cells, NK cells, NKT cells, macrophages and dendritic cells. Thus, the types of compounds discussed above can cause an increase in the type I immune responses in the immune system. As a result, the compounds discussed and disclosed herein would clearly provide a benefit in the treatment, prevention, or amelioration of macular degeneration. The effects of the types of compounds discussed above can be evaluated by simple blood concentration of soluble mediators that are mentioned above in the treated subjects upon sufficient duration of exposure of the compounds.

In first set of study, 2-[4-(7-Ethyl-5H-pyrrolo[2,3-b]pyrazin-6-yl)-phenyl]-propan-2-ol was tested at 3, 10, and 30 mpk dose and 2-(3-fluoro-phenyl)-4-methyl-pyrimidine-5-carboxylic acid indol-1-ylamide (hPGDS inhibitor) was tested at 10 mpk. PO treatment of rats with 3, 10, and 30 mpk of 2-[4-(7-ethyl-5H-pyrrolo[2,3-b]pyrazin-6-yl)-phenyl]-propan-2-ol
resulted in reduction of CNV plaque thickness of 11.3%, 36.6%, and 32.2%, respectively. In addition, PO treatment of 2-(3-fluoro-phenyl)-4-methyl-pyrimidine-5-carboxylic acid indol-1-ylamide at 10 mpk resulted in 40.7% decrease in CNV plaque thickness as shown in FIG. 1. This was better than the positive control, intravitreal injection of Triamcinolone Acetonide, one of current standard of care, which resulted in 25.9% reduction of CNV plaque thickness.

In second set of study, 2-[4-(7-Ethyl-5H-pyrrolo[2,3-b]pyrazin-6-yl)-phenyl]-propan-2-ol was tested at 1.25, 6, and 30 mpk dose, PO, BID. In addition, Phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt and 2-(3-{6-[2-(2,4-dichloro-phenyl)-ethylamino]-2-methoxy-pyrimidin-4-yl}-phenyl)-2-methyl-propionic acid phosphoric acid salt were tested at 30 mpk and 12.5 mpk, with same regimen, respectively. Although we have yet to calculate the CNV plaque thickness from the histology slides, preliminary data of florescent angiography pictures suggest there is a significant efficacy for all compounds tested. The pictures and legends are provided in FIGs 2 and 3.

The data collected also clearly shows that these compounds evaluated in the rat LIMD model demonstrated significant reduction of CNV formation measured by the thickness of the choroid neovascular plaque formation by histological analyses as well as retina florescent angiography.

The data validate that these types of compounds readily have applications for treatment of wet AMD. Moreover, these compounds are clearly useful in a novel method of the present invention for treating wet AMD, and for arresting the progression of AMD from dry form (geographic atrophy) to the wet form. The data also show that compounds such as tryptase beta inhibitors or other key mast cell inhibitors can be beneficial in the treatment of AMD. An example of such a tryptase beta inhibitor having applications in a method of the present invention is 4-(5-aminomethyl-2-fluoro-phenyl)-piperidin-1-yl]-[7-fluoro-1-(2-methoxy-ethyl)-4-trifluorometoxy-1H-indol-3-yl]-methanone (shown below),

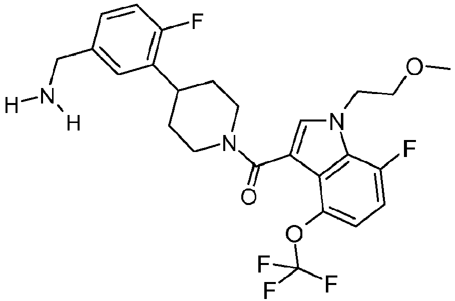

Other examples of tryptase beta inhibitors are set forth in US Patent 6,977,283 and PCT published application WO 2005/097780, which are hereby incorporated by reference herein in their entireties. In addition, the data from DP antagonist indicate that altered activation ofNK, NKT cells as well as dendritic cells can be a valid approach for the treatment of the AMD.

The present invention is not to be limited in scope by the specific embodiments describe herein. Indeed, various modifications of the invention in addition to those described herein will become apparent to those skilled in the art from the foregoing description and the accompanying figures. Such modifications are intended to fall within the scope of the appended claims.

The invention is summarized as follows:
1. A method for preventing, treating or ameliorating choroidal neovascularization in a patient comprising, administering to the patient an effective amount of a compound that modulates the patient's immune system, wherein the immune system has a type I immune responses and a type II immune responses, such that administering the compound to the patient increases the type I immune responses in the patient as compared to the type I immune responses in the patient prior to administering of the compound.
2. The method of Item 1, wherein the compound is selected from the group consisting of:
   (a) a syk multikinase inhibitor;
   (b) an hPGDS inhibitor; and
   (c) a DP antagonist.
3. The method of Item 2, wherein the syk multikinase inhibitor is selected from the group consisting of phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester acetic acid salt 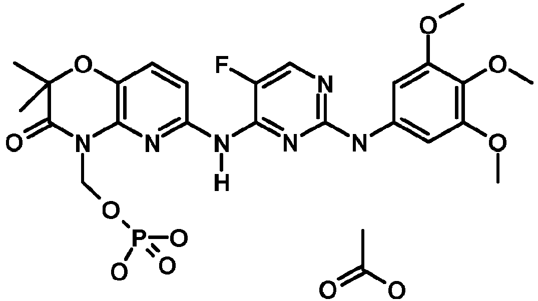 and 2-[4-(7-ethyl-5H-pyrrolo[2,3-b]pyrazin-6-yl)-phenyl]-propan-2-ol 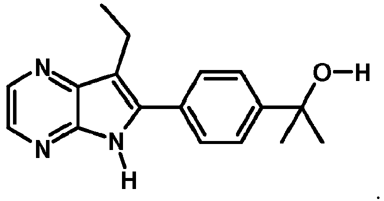
4. The method of Item 2, wherein the hPGDS inhibitor is selected from the group consisting of:
   2-(3-fluoro-phenyl)-4-methyl-pyrimidine-5-carboxylic acid indol-1-ylamide 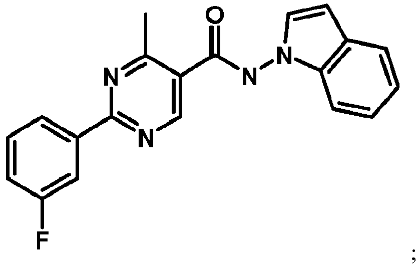
   2-Pyridin-2-yl-pyrimidine-5-carboxylic acid 3-[5-(1-hydroxy-1-methyl-ethyl)-1,2,4-oxadiazol-3-yl]-benzylamide 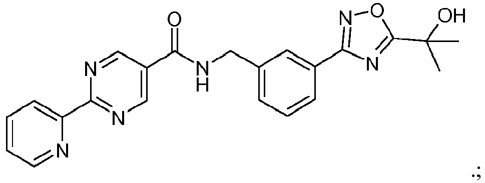 and
   4-Methyl-2-pyridin-2-yl-pyrimidine-5-carboxylic acid (5-fluoro-3-methyl-indol-1-yl)-amide 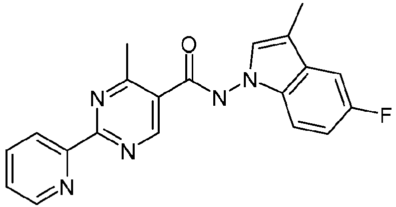
5. The method of Item 2, wherein the DP antagonist is 2-(3-{6-[2-(2,4-dichloro-phenyl)-ethylamino]-2-methoxy-pyrimidin-4-yl}-phenyl)-2-methyl-propionic acid phosphoric acid salt 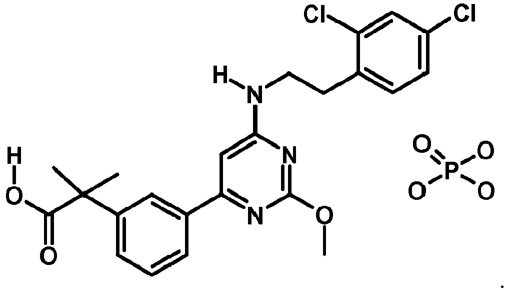
6. The method of any of Items 1-5, wherein the compound modulates the activity of an immunocyte in the patient.
7. The method of Item 6, wherein the immunocyte is a natural killer cell (NK cell), a natural killer T cell (NKT cell), a mast cell, a dendritic cell, a granulocyte selected from the group consisting of an eosinophil, a basophil and neutrophil, or any combination thereof.
8. The method of any of Items 1-7, wherein the preventing, treating or ameliorating choroidal neovascularization also prevents, treats or ameliorates wet macular degeneration in the patient.
9. The method of any of Items 1-7, wherein the preventing, treating or ameliorating choroidal neovascularization also prevents, treats or ameliorates age-related macular degeneration in the patient.
10. A method for treating, ameliorating or preventing macular degeneration in a patient, comprising administering to the patient an effective amount of a compound that modulates the patient's immune system, wherein the immune system has a type I immune responses and a type II immune responses, such that administering the compound to the patient increases the type I immune responses in the patient as compared to the type I immune responses in the patient prior to administering of the compound.
11. The method of Item 10, wherein the macular degeneration in the patient is age-related macular degeneration.
12. The method of either of Items 10 or 11, wherein the compound modulates the activity of an immunocyte in the patient.
13. The method of Item 12, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof.
14. The method of any of Items 10-13, wherein the compound is selected from the group consisting of:
   (a) a syk multikinase inhibitor;
   (b) an hPGDS inhibitor; and
   (c) a DP antagonist.
15. The method of Item 14, wherein the syk multikinase inhibitor is phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester acetic acid salt 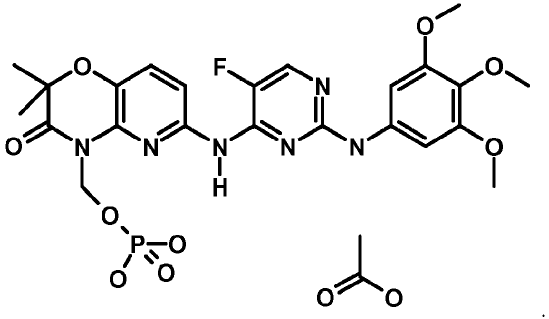
16. The method of Item 14, wherein the hPGDS inhibitor is selected from the group consisting of: 2-(3-fluoro-phenyl)-4-methyl-pyrimidine-5-carboxylic acid indol-1-ylamide 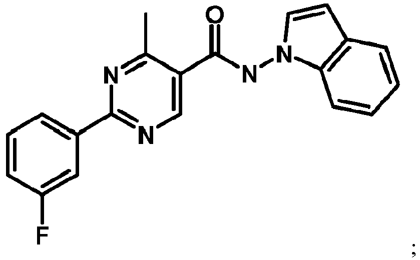 2-Pyridin-2-yl-pyrimidine-5-carboxylic acid 3-[5-(1-hydroxy-1-methyl-ethyl)-1,2,4-oxadiazol-3-yl]-benzylamide 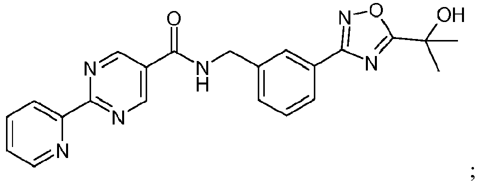 and
   4-Methyl-2-pyridin-2-yl-pyrimidine-5-carboxylic acid (5-fluoro-3-methyl-indol-1-yl)-amide 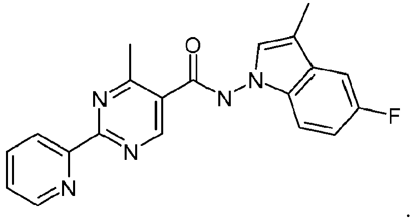
17. The method of Item 14, wherein the DP antagonist is 2-(3-{6-[2-(2,4-dichloro-phenyl)-ethylamino]-2-methoxy-pyrimidin-4-yl}-phenyl)-2-methyl-propionic acid phosphoric acid salt 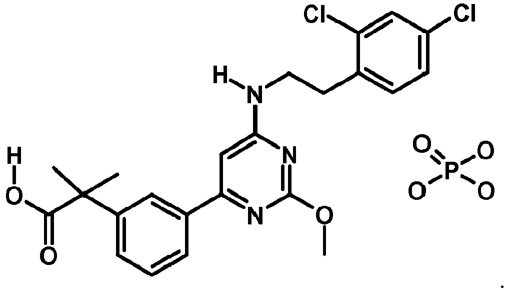
18. A method for preventing, treating or ameliorating macular degeneration in a patient, comprising administering to the patient an effective amount of a compound that modulates the patient's immune system, wherein the immune system has a type I immune responses and a type II immune responses, such that administering the compound to the patient increases the type I immune responses in the patient as compared to the type I immune responses in the patient prior to administering of the compound.
19. The method of Item 18, wherein the macular degeneration in the patient is age-related macular degeneration.
20. The method of either of Items 18 or 19, wherein the administration of the compound also prevents, treats or ameliorates choroidal neovascularization in the patient.
21. The method of any of Items 18-20, wherein the compound modulates the activity of an immunocyte in the patient.
22. The method of Item 21, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof.
23. The method of any of Items 18-22, wherein the compound is selected from the group consisting of:
   (a) a syk multikinase inhibitor;
   (b) an hPGDS inhibitor; and
   (c) a DP antagonist.
24. The method of Item 23, wherein the syk multikinase inhibitor is phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester acetic acid salt 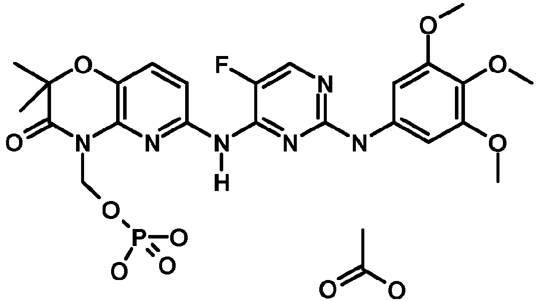
25. The method of Item 23, wherein the hPGDS inhibitor is selected from the group consisting of:
   2-(3-fluoro-phenyl)-4-methyl-pyrimidine-5-carboxylic acid indol-1-ylamide 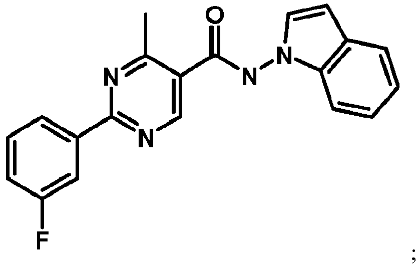
   2-Pyridin-2-yl-pyrimidine-5-carboxylic acid 3-[5-(1-hydroxy-1-methyl-ethyl)-1,2,4-oxadiazol-3-yl]-benzylamide 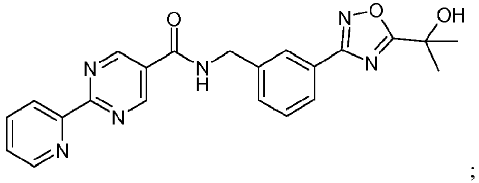 and
   4-Methyl-2-pyridin-2-yl-pyrimidine-5-carboxylic acid (5-fluoro-3-methyl-indol-1-yl)-amide 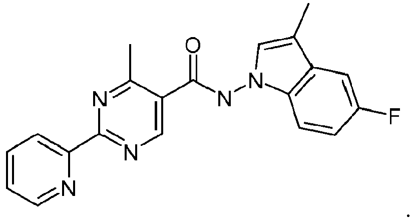
26. The method of Item 23, wherein the DP antagonist is 2-(3-{6-[2-(2,4-dichloro-phenyl)-ethylamino]-2-methoxy-pyrimidin-4-yl}-phenyl)-2-methyl-propionic acid phosphoric acid salt 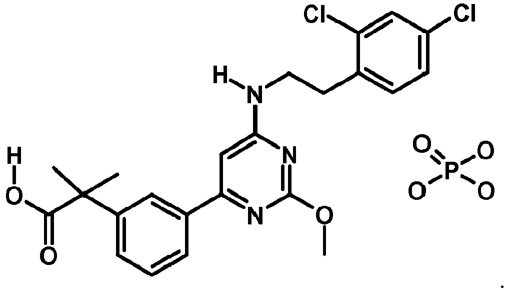
27. A method of treating, preventing or ameliorating macular degeneration in a patient having an immune system which has a type I immune responses and a type II immune responses, the method comprising administering to the patient an effective amount of a compound that modulates the activity of an immunocyte in the patient, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof, which increases the type I immune responses in the patient relative to the type I immune responses in the patient prior to administering the compound, and the increase in the type I responses treats, ameliorates or prevents choroidal neovascularization in the patient.
28. The method of Item 27, wherein the macular degeneration is age-related macular degeneration.
29. The method of either of Item 27 or 28, wherein the compound is selected from the group consisting of:
   (a) a syk multikinase inhibitor;
   (b) an hPGDS inhibitor; and
   (c) a DP antagonist.
30. The method of Item 29, wherein the syk multikinase inhibitor is selected from the group consisting of:
   phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt; and 2-[4-(7-ethyl-5H-pyrrolo[2,3-b]pyrazin-6-yl)-phenyl]-propan-2-ol.
31. The method of Item 29, wherein the hPGDS inhibitor is selected from the group consisting of:
   2-(3-fluoro-phenyl)-4-methyl-pyrimidine-5-carboxylic acid indol-1-ylamide;
   2-Pyridin-2-yl-pyrimidine-5-carboxylic acid 3-[5-(1-hydroxy-1-methyl-ethyl)-1,2,4-oxadiazol-3-yl]-benzylamide; and
   4-Methyl-2-pyridin-2-yl-pyrimidine-5-carboxylic acid (5-fluoro-3-methyl-indol-1-yl)-amide.
32. The method of Item 29, wherein the DP antagonist is 2-(3-{6-[2-(2,4-dichloro-phenyl)-ethylamino]-2-methoxy-pyrimidin-4-yl}-phenyl)-2-methyl-propionic acid phosphoric acid salt.
33. A method of preventing, treating or ameliorating wet macular degeneration in a patient having an immune system which has a type I immune responses and a type II immune responses, the method comprising administering to the patient an effective amount of a compound that modulates the activity of an immunocyte in the patient, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof, so that the type I immune responses in the patient are increased relative to the type I immune responses prior to administering the compound, and the increase in the type I responses ameliorates or prevents choroidal neovascularization in the patient, wherein the compound is selected from the group consisting of:
   (a) phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt;
   (b) 2-[4-(7-ethyl-5H-pyrrolo[2,3-b]pyrazin-6-yl)-phenyl]-propan-2-ol;
   (c) 2-(3-fluoro-phenyl)-4-methyl-pyrimidine-5-carboxylic acid indol-1-ylamide;
   (d) 2-(3-{6-[2-(2,4-dichloro-phenyl)-ethylamino]-2-methoxy-pyrimidin-4-yl}-phenyl)-2-methyl-propionic acid phosphoric acid salt;
   (e) 2-Pyridin-2-yl-pyrimidine-5-carboxylic acid 3-[5-(1-hydroxy-1-methyl-ethyl)-1,2,4-oxadiazol-3-yl]-benzylamide; and
   (f) 4-Methyl-2-pyridin-2-yl-pyrimidine-5-carboxylic acid (5-fluoro-3-methyl-indol-1-yl)-amide.
34. A method of preventing, treating or ameliorating macular degeneration in a patient having an immune system which has a type I immune responses and a type II immune responses, the method comprising administering to the patient an effective amount of a compound that modulates the activity of an immunocyte in the patient, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof, so that the type I immune responses in the patient are increased relative to the type I immune responses prior to administering the compound, and the increase in the type I responses treats, ameliorates or prevents choroidal neovascularization in the patient, wherein the compound is selected from the group consisting of:
   (a) phosphoric acid mono-{6-[5-fluoro-2-(3,4,5-trimethoxy-phenylamino)-pyrimidin-4-ylamino]-2,2-dimethyl-3-oxo-2,3-dihydro-pyrido[3,2-b][1,4]oxazin-4-ylmethyl} ester Acetic Acid Salt;
   (b) 2-[4-(7-ethyl-5H-pyrrolo[2,3-b]pyrazin-6-yl)-phenyl]-propan-2-ol;
   (c) 2-(3-fluoro-phenyl)-4-methyl-pyrimidine-5-carboxylic acid indol-1-ylamide;
   (d) 2-(3-{6-[2-(2,4-dichloro-phenyl)-ethylamino]-2-methoxy-pyrimidin-4-yl}-phenyl)-2-methyl-propionic acid phosphoric acid salt;
   (e) 2-Pyridin-2-yl-pyrimidine-5-carboxylic acid 3-[5-(1-hydroxy-1-methyl-ethyl)-1,2,4-oxadiazol-3-yl]-benzylamide; and
   (f) 4-Methyl-2-pyridin-2-yl-pyrimidine-5-carboxylic acid (5-fluoro-3-methyl-indol-1-yl)-amide.

## Claims

1. A DP antagonist that modulates a patient's immune system for use in preventing, treating or ameliorating choroidal neovascularisation and/or macular degeneration in the patient, wherein the immune system has type I immune responses and type II immune responses, such that administering the DP antagonist to the patient increases the type I immune responses in the patient as compared to the type I immune responses in the patient prior to administering of the DP antagonist.

2. The DP antagonist for use of claim 1, wherein the DP antagonist is 2-(3- {6-[2-(2,4-dichlorophenyl)-ethylamino]-2-methoxy-pyrimidin-4-yl}-phenyl)-2-methyl-propionic acid phosphoric acid salt

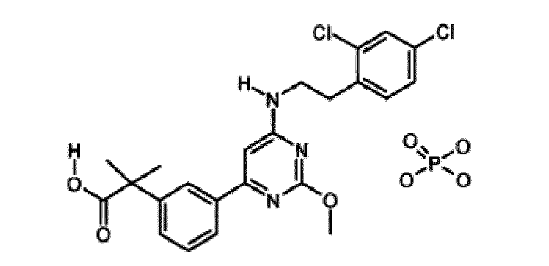

3. The DP antagonist for use of any of claims 1-2, wherein the DP antagonist modulates the activity of an immunocyte in the patient.

4. The DP antagonist for use of claim 3, wherein the immunocyte comprises or is a natural killer cell (NK cell), a natural killer T cell (NKT cell), a mast cell, a dendritic cell, a granulocyte selected from the group consisting of an eosinophil, a basophil and neutrophil, or any combination thereof.

5. The DP antagonist for use of any of claims 1-4, wherein the macular degeneration is selected from the group consisting of dry macular degeneration, wet macular degeneration, and age-related macular degeneration.

6. The DP antagonist for use of any of claims 1-4, wherein the preventing, treating or ameliorating choroidal neovascularization also prevents, treats or ameliorates macular degeneration or wet macular degeneration in the patient.

7. The DP antagonist for use of any of claims 1-4, wherein the preventing, treating or ameliorating choroidal neovascularization also prevents, treats or ameliorates age-related macular degeneration in the patient.

8. A DP antagonist that modulates the activity of an immunocyte in a patient for use in treating, preventing or ameliorating macular degeneration in the patient having an immune system which has type I immune responses and type II immune responses, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof, wherein administering the DP antagonist increases the type I immune responses in the patient relative to the type I immune responses in the patient prior to administering the DP antagonist, and the increase in the type I responses treats, ameliorates or prevents choroidal neovascularization in the patient.

9. A DP antagonist that modulates the activity of an immunocyte in a patient for use in preventing, treating or ameliorating wet macular degeneration in the patient having an immune system which has type I immune responses and type II immune responses, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof, so that by administering the DP antagonist the type I immune responses in the patient are increased relative to the type I immune responses prior to administering the DP antagonist, and the increase in the type I responses ameliorates or prevents choroidal neovascularization in the patient, wherein the DP antagonist is 2-(3-{6-[2-(2,4-dichloro-phenyl)-ethylamino]-2-methoxy-pyrimidin-4-yl}-phenyl)-2-methyl-propionic acid phosphoric acid salt.

10. A DP antagonist that modulates the activity of an immunocyte in a patient for use in preventing, treating or ameliorating macular degeneration in the patient having an immune system which has type I immune responses and type II immune responses, wherein the immunocyte comprises a natural killer cell (NK cell), a natural killer T cell (NKT cell), a mast cell, a dendritic cell, a granulocyte selected from the group consisting of an eosinophil, a basophil and a neutrophil, or any combination thereof, so that by administering the DP antagonist the type I immune responses in the patient are increased relative to the type I immune responses prior to administering the DP antagonist, and the increase in the type I responses treats, ameliorates or prevents choroidal neovascularization in the patient, wherein the DP antagonist is 2-(3-{6-[2-(2,4-dichloro-phenyl)-ethylamino]-2-methoxy-pyrimidin-4-yl}-phenyl)-2-methyl propionic acid phosphoric acid salt.
